# EUROPÄISCHE PATENTANMELDUNG

(11) **EP 0 870 511 A2**
(43) Veröffentlichungstag der Anmeldung: **14.10.1998**
(21) Anmeldenummer: 98106005.6
(22) Anmeldetag: 02.04.1998
(51) Int. Cl.: A61L 2/06, A61L 2/26

(54) **Wasser-Vorratsbehälter in einer Vorrichtung zum Spülen und/oder Desinfizieren von Pflegegeschirren**

(30) Priorität: 12.04.1997 DE 29706596 U; 14.10.1997 DE 29718110 U
(71) Anmelder: Discher Sanitätstechnik GmbH, 42781 Haan (DE)
(72) Erfinder: Discher, Josef, 42781 Haan (DE)
(74) Vertreter: Grundmann, Dirk, Dr.

(57) **Zusammenfassung**

Die Erfindung betrifft einen Wasser-Vorratsbehälter (1) in einer Vorrichtung zum Spülen und/oder Desinfizieren von Pflegegeschirren, insbesondere Bett-Pflegegeschirr, mit einer das Pflegegeschirr aufnehmenden Kammer, welcher der Wasser-Vorratsbehälter (1) zulaufseitig vorgeordnet ist und mit einem mit dem Wasser des Wasser-Vorratsbehälters (1) gespeisten Dampferzeuger (3). Zwecks Realisierung eines raumsparenden Aufbaues und eines hohen Desinfektionsgrades schlägt die Erfindung vor, daß der Dampferzeuger (3) integraler Bestandteil des Wasser-Vorratsbehälters (1) ist.

## Beschreibung

Die Erfindung betrifft einen Wasser-Vorratsbehälter in einer Vorrichtung zum Spülen und/oder Desinfizieren von Pflegegeschirren, insbesondere Bett-Pflegegeschirr, mit einer das Pflegegeschirr aufnehmenden Kammer, welcher der Wasser-Vorratsbehälter zulaufseitig vorgeordnet ist und mit einem mit dem Wasser des Wasser-Vorratsbehälters gespeisten Dampferzeuger.

Eine Vorrichtung der in Rede stehenden Art ist bekannt aus dem DE-GM 92 15 876, wobei zur Erzielung einer dezentralen thermischen Desinfektion der Dampferzeuger getrennt von dem Wasser-Vorratsbehälter angeordnet und von diesem gespeist ist. Nach beendigtem Spülvorgang des Pflegegeschirrs wird dann der Dampf in die Spülkammer zur Desinfektion geleitet.

Dem Gegenstand der Erfindung liegt die Aufgabe zugrunde, einen Wasser-Vorratsbehälter der in Rede stehenden Art von erhöhtem Gebrauchswert anzugeben.

Diese Aufgabe ist zunächst und im wesentlichen bei einem Wasser-Vorratsbehälter mit den Merkmalen des Anspruchs 1 gelöst, wobei darauf abgestellt ist, daß der Dampferzeuger integraler Bestandteil des Wasservorratsbehälters ist.

Die Unteransprüche betreffen vorteilhafte Weiterbildungen der erfindungsgemäßen Lösung.

Zufolge derartiger Ausgestaltung ist ein gattungsgemäßer Wasser-Vorratsbehälter von erhöhtem Gebrauchswert geschaffen. Er erfüllt nun eine Doppelfunktion:

Einerseits bevorratet er das der Kammer zuzuleitende Spülwasser; andererseits beinhaltet er den Dampferzeuger, so daß neben dem Vorteil einer Raumeinsparung noch derjenige einer Kosteneinsparung erzielt wird. Weiterhin ist nicht unerheblich, daß die Ausbildung des Dampferzeugers als integraler Bestandteil des Wasservorratsbehalters dieser stets durch die Dampfentwicklung im Dampferzeuger desinfiziert wird verbunden damit, daß jegliche Ansiedlung von Bakterien im Inneren des Wasservorratsbehälters mit Sicherheit vermieden ist. Wenn der Dampferzeuger bodenseitig des Wasservorratsbehälters angeordnet ist, wird gleichzeitig mit dem Befüllen des Wasservorratsbehälters zuvor der Dampferzeuger mit Wasser beliefert. Es ist daher stets gewährleistet, daß beim Einschalten des Dampferzeugers die nötige Flüssigkeitsmenge im Dampferzeuger vorhanden ist. In einfacher Weise ist dabei der Dampferzeuger als bodenseitiger Fortsatz gestaltet. Er beinhaltet das entsprechende Volumen, welches für die thermische Desinfektion bei einem Spülvorgang mit anschließender Desinfektion benötigt wird. Damit keine Beeinträchtigung der Wasservorratsmenge innerhalb des Dampferzeugers bei Wasserentnahme aus dem Vorratsbehälter beim Spülvorgang stattfindet, liegt die Ablauföffnung des Wasser-Vorratsbehälters auf einer das Füllniveau des Dampferzeugers definierenden Höhe. Dagegen befindet sich die Dampfaustrittsöffnung im oberen oder Deckelbereich des Wasser-Vorratsbehälters. Das bedeutet, daß der aus dem Dampferzeuger austretende Dampf vor Verlassen des Wasservorratsbehälters auch dessen oberen Bereich desinfiziert. Der Austritt von Dampf aus der Wasserzulauföffnung ist dadurch verhindert, daß die Wasserzulauföffnung des Deckels einen Dampfaustritts-Widerstand besitzt. Das Wasser seinerseits kann dagegen ungehindert in das Innere des Wasservorratsbehälters gelangen. Dies wird in einfacher Weise durch eine auf die Wasserzulauföffnung gerichtete Wasserstrahl-Austrittsmündung einer Wasserversorgungsleitung und ein die Wasserzulauföffnung verschließendes Rückschlagventil erreicht, welches durch den Strahldruck öffenbar ist. In einfacher Weise ist dabei so vorgegangen, daß das Rückschlagventil als Gemmizunge gestaltet ist. Beim Zulauf des Wassers in den Vorratsbehälter verschwenkt die einseitig festgelegte Gummizunge in die Öffnungsstellung. Wird der Dampferzeuger eingeschaltet, ist demgemäß auch der Wasserzulauf gestoppt, so bewirkt die Dampfentwicklung einen im Wasser-Vorratsbehälter zunehmenden Druck, durch welchen die Gemmizunge in ihre Dichtstellung gebracht wird, so daß der Dampf ausschließlich durch die Dampfaustrittsöffnung den Wasser-Vorratsbehälter verlassen kann. Schließlich besteht ein vorteilhaftes Merkmal noch darin, daß der Dampferzeuger einen Wasseraufnahmeraum ausbildet mit bodenseitiger Heizwendel, mit einem etwa auf halber Höhe des Wasseraufhahmeraunes oberhalb der Heizwendel angeordneten Temperaturfühler. Auf diese Weise wird eine Überhitzung vermieden. Fällt der Niveauspiegel innerhalb des Wasseraufnahmeraumes unter den Temperaturfühler, so vermeldet dieser den Zustand und stellt die Stromzufuhr zur Heizwendel ab, so daß Gewähr gegeben ist, daß nur bei von Wasser umgebener Heizwendel das Aufheizen zur Dampferzeugung erfolgt.

Eine alternative Lösung ist darin zu sehen, daß der Dampfaustritts-Widerstand von einem syphonartigen Verschluß ausgebildet ist. Ein gesondertes Ventil kann demgemäß entfallen. Hierdurch vereinfacht sich der Aufbau. Dennoch arbeitet diese Ausgestaltung sehr wirkungsvoll. Im Detail ist der syphonartige Verschluß in der Weise ausgebildet, daß er ein vom Deckel bis unmittelbar über den Boden reichendes Tauchrohr aufweist, welches in der Dampferzeugungsphase in den Wasserspiegel eines im Behälter verbleibenden Restwasservolunens eintaucht. Zwecks einer Dampferzeugung wird der Vorratsbehälter so weit geleert, daß das Tauchrohr stets noch in das Restwasservolumen eingetaucht bleibt. Sodann besitzt der Dampferzeuger einen sich oberhalb des Behälterbodens erstreckenden Innenbehälter. Dieser befindet sich demgemäß in einer geschützten Lage. Die Anordnung des Innenbehälters ist derart, daß der Boden des Innenbehälters oberhalb des Wasserspiegels des Restwasservolunens liegt. Das bedeutet, daß in der Dampferzeugungsphase das Restwasservolumen weitgehend nicht miterhitzt wird. Weiterhin ist erfindungsgemäß vorgesehen, daß die Wandungen des Innenbehälters ein Überlaufniveau überragen. Demgemäß kann in der Dampferzeugungsphase kochendes Wasser nicht über die Behälterwände hinausspritzen, so daß das im Innenbehälter befindliche Wasser vollständig in Dampf umgewandelt wird. In einfacher Weise ist dabei das Überlaufniveau durch die Mündungsöffnung eines U-förmigen Hebers definiert. Wird zum Zwecke der Dampferzeugrnng und der damit verbundenen Desinfektion der Vorratsbehälter bis auf das Restwasservolumen geleert, so erfolgt eine Leerung des Innenbehälters bis auf Höhe der Mündungsöffnung des U-förmigen Hebers. In vorteilhafter Weise ist diesbezüglich vorgesehen, daß der U-Scheitel unterhalb der Innenbehälterwand-Oberkante liegt. Im Detail sieht dies so aus, daß das Überlaufniveau etwa auf der Hälfte der Innenbehälterhöhe liegt, so daß, wie vorerwähnt wurde, das im Innenbehälter verbleibende Wasser vollständig in Dampf umgewandelt wird. Eine baulich günstige Maßnahme ist darin zu sehen, daß der Temperaturfühler von oben in den Innenbehälter ragt. Die sowieso vorhandene obere Öffnung ist daher zum Eintritt des Temperaturfühlers ausgenutzt. Er braucht keine Seitenwände zu durchgreifen, was ebenfalls zu einer kostensenkenden Ausgestaltung führt. Wartungstechnisch ist es von Vorteil, eine handverschließbare Inspektionsöffnung im oberen Bereich, insbesondere im Bereich des Wasserspiegels des Vorratsbehälters, vorzusehen. Die Inspektionsöffnung kann dabei durch einen Schraubstopfen verschließbar sein. Eine großdimenzionierte Heizwendel kann dadurch realisiert werden, daß die Länge des Innenbehälters nur geringfügig kleiner ist als die Innenweite des Vorratsbehälters. Diese große Länge des Innenbehälters ist andererseits ausgenutzt, eine flache, langgestreckte Heizwendel einzusetzen verbunden mit dem Vorteil eines schnellen Aufheizens des im Innenbehälter verbleibenden Wassers, was wiederum zu verkürzten Desinfizierungszeiten führt. Schließlich ist ein vorteilhaftes Merkmal noch darin zu sehen, daß die Wandungen des Innenbehälters bis auf eine Wandung beabstandet zu den Wänden des Vorratsbehälters angeordnet sind. Die eine Wandung dient dabei zur Festlegung des Innenbehälters an dem Vorratsbehälter selbst.

Nachstehend werden zwei Ausführungsbeispiele der Erfindung anhand der Zeichnungen erläutert. Es zeigt
- Fig. 1: einen Längsschnitt durch einen erfindungsgemäßen Wasser-Vorratsbehälter, betreffend die erste Ausführungsform,
- Fig. 2: die Ansicht gemäß Pfeilrichtung II in Fig. 1, teilweise aufgebrochen dargestellt,
- Fig. 3: eine Draufsicht auf den Wasser-Vorratsbehälter,
- Fig. 4: den Schnitt nach der Linie IV-IV in Fig. 1,
- Fig. 5: teils in Ansicht, teils im Längsschnitt einen Wasser-Vorratsbehälter gemäß der zweiten Ausführungsform,
- Fig. 6: die dicht in Pfeilrichtung VI in Fig. 5 und
- Fig. 7: eine der Fig. 5 entsprechende Darstellung, wobei der Wasser-Vorratsbehälter bis auf ein Restwasservolumen geleert ist, in welches das untere Ende eines Tauchrohres eintaucht.

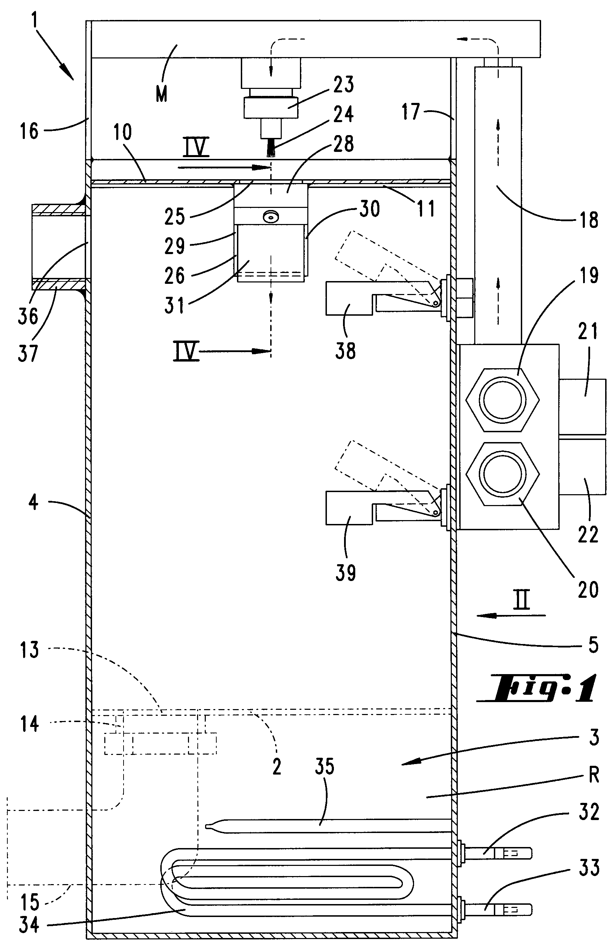

Gemäß der ersten Ausführungsform ist mit der Ziffer 1 als Ganzes ein Wasser-Vorratsbehälter bezeichnet. Er besitzt rechteckigen Grundriß. Vom Behälterboden 2 geht abwärts gerichtet ein kammerartiger bodenseitiger Fortsatz aus, welcher einen Dampferzeuger 3 bildet. Dieser erstreckt sich über die gesamte Länge zwischen zwei parallel zueinander angeordneten Behälter-Seitenwänden 4 und 5, vergl. Fig. 1. Die eine Seitenwand 6 des Dampferzeugers 3 geht ansatzlos über in die Behälter-Seitenwand 7, während die der Seitenwand 6 gegenüberliegende Seitenwand 8 des Dampferzeugers 3 parallel zur Behälter-Seitenwand 9 verläuft und in einem solchen Abstand zu dieser angeordnet ist, welcher etwas größer ist als die Hälfte des Abstandes zwischen den Behälter-Seitenwänden 7, 9.

Oberseitig ist der Wasser-Vorratsbehälter 1 durch einen einlegbaren Deckel 10 verschlossen. Dieser liegt auf den horizontalen Schenkein 11 von Winkelleisten 12 auf, die ihrerseits innenseitig der Behälter-Seitenwände 7, 9 am oberen Ende derselben vorzugsweise durch Punktschweißung festgelegt sind.

Der Behälterboden 2 bildet eine Ablauföffnung 13 mit zugehörigem Anschlußstutzen 14 aus. Dieser dient zum Anschluß einer Leitung 15, welche zu einer nicht veranschaulichten Druckerhöhungspumpe führt, von welcher eine ebenfalls nicht veranschaulichte Kammer zur Aufnahme des Bett-Pflegegeschirrs gespeist wird. Dies ist bekannter Stand der Technik, so daß darauf nicht näher eingegangen wird. Aufgrund der Ablauföffnung 13 im Behälterboden 2 erstreckt sich das Flüssigkeitsniveau innerhalb des Dampferzeugers 3 auf Höhe des Behälterbodens 2.

Von den oberen Stirnseiten der Behälter-Seitenwände 4, 5 gehen mittig Stege 16, 17 aus, welche mit Abstand zum Deckel 10 ein im Querschnitt rechteckiges Mischrohr M tragen. Dessen eines Ende schließt bündig mit dem Steg 16 ab, während das andere Ende den Steg 17 überragt und dort mit einer Zulaufleitung 18 verbunden ist. Diese verläuft etwa parallel zur Behälter-Seitenwand 5 und steht ihrerseits in Leitungsverbindung mit zwei übereinander angeordneten Zulaufstutzen 19, 20 für Kalt- und Warmwasser, welchen Zulaufstutzen 19, 20 je ein eigenes Magnetventil 21 bzw. 22 zugeordnet ist.

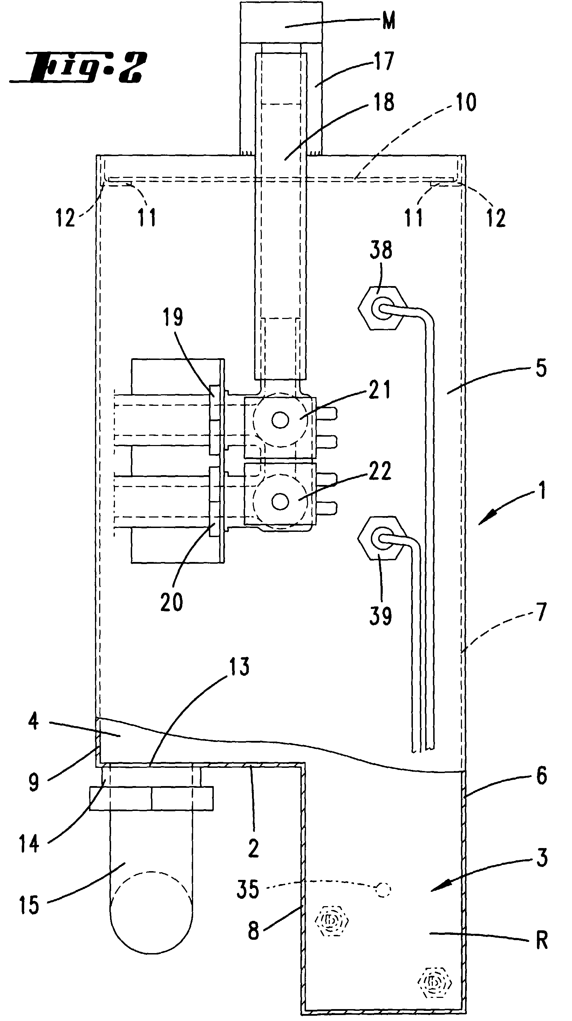

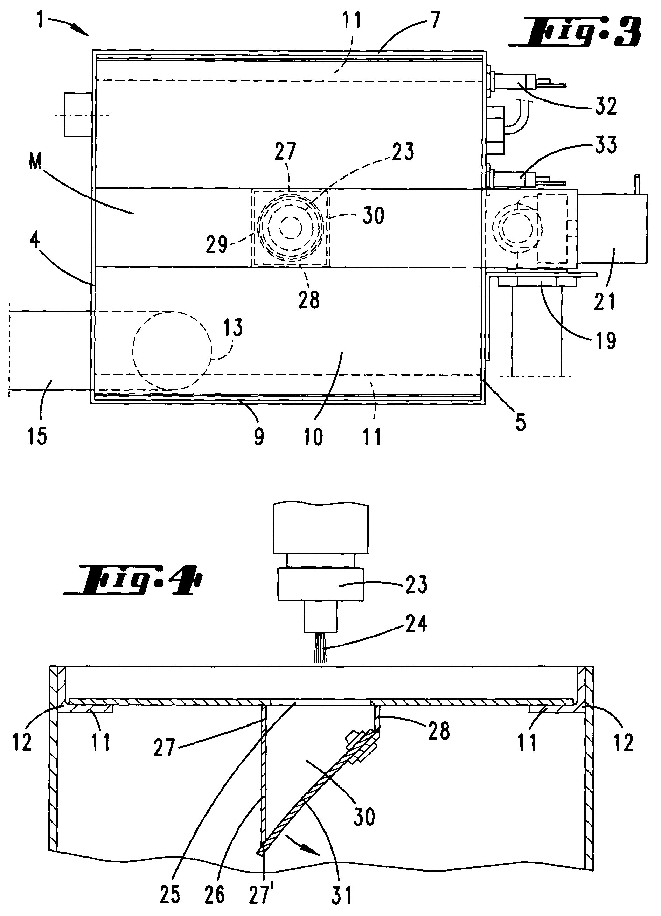

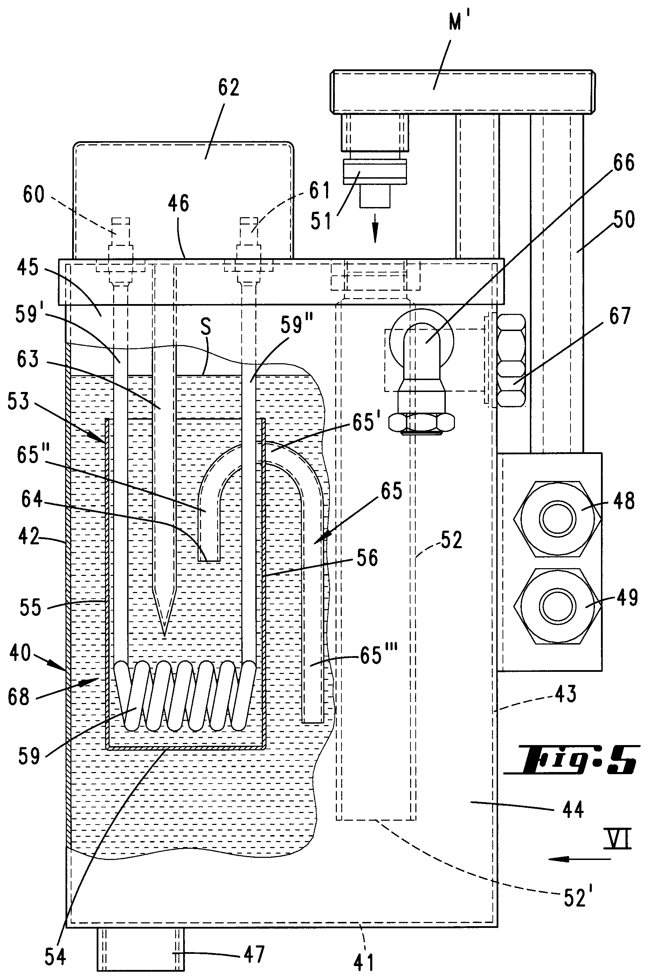

Das Mischrohr M trägt unterseitig ein Ausströmventil 23 mit im Bereich der Strahlaustrittsöffnung angeordneten, nicht veranschaulichten Strahlleitkörpern zur Bildung eines Wasserstrahls 24. Sodann erstreckt sich das Ausströmventil 23 mit Abstand oberhalb einer Wasserzulauföffnung 25 des Deckels 10. Die Wasserzulauföffnung 25 des Deckels 10 besitzt einen Dampfaustritts-Widerstand. Hierzu setzt sich der Deckel 10 behältereinwärts, die Wasserzulauföffnung 25 flankierend, in ein im Grundriß quadratisches Ventilgehäuse 26 fort. Zwei sich gegenüberliegende Gehäuzewände 27, 28 sind unterschiedlich lang bemessen verbunden damit, daß die sie verbindenden Gehäusewände 29, 30 trapezförmig verlaufen. Die kürzere Gehäusewand 28 ist Träger einer schräg abwärts gerichteten Gummizunge 31, welche dichtend zwischen den Gehäusewänden 29, 30 verläuft und gegen die Unterkante 27' der längeren Gehäusewand 27 tritt, vergl. Fig. 4. Auf diese Weise ist ein die Wasserzulauföffnung 25 verschließendes Rückschlagventil geschaffen, welches durch den aus dem Ausströmventil 23 austretenden Strahldruck öffnet. Hierdurch bildet das als Gummizunge 31 ausgebildete Rückschlagventil den Dampfaustritts-Widerstand im Bereich der Wasserzulauföffnung 25.

Die sich über den Behälterboden 2 fortsetzende Behälter-Seitenwand 5 trägt in ihrem den Wasseraufnahmeraum R bildenden Bereich die elektrischen Anschlüsse 32, 33 für eine in den Wasseraufnahmeraum R hineinragende Heizwendel 34. Wie Fig. 1 veranschaulicht, erstreckt sich diese im unteren Bereich des Wasseraufnahmeraumes R. Etwa auf halber Höhe des Wasseraufnanmeraumes R ist oberhalb der Heizwendel 34 ein Temperaturfühler 35 angeordnet.

Weiterer Bestandteil des Wasser-Vorratsbehälters 1 ist eine Dampfaustrittsöffnung 36. Dieselbe kann sich im oberen oder Deckelbereich des Wasservorratsbehälters 1 befinden. Beim Ausführungsbeispiel erstreckt sich die Dampfaustrittsöffnung 36 am oberen Ende der Behälter-Seitenwand 4. Außenseitig trägt letztere einen die Dampfaustrittsöffnung 36 flankierenden Anschlußstutzen 37.

Sodann sind innenseitig der Behälter-Seitenwand 5 zwei Niveauhöhenregler 38, 39 vorgesehen.

Es stellt sich folgende Wirkungsweise ein. Zwecks Durchführen einer Reinigung des Bettgeschirrs innerhalb der Kammer werden die Magnetventile 21, 22 der Zulaufstutzen 19, 20 gleichzeitig oder nacheinander geöffnet, so daß das Wasser über die Zulaufleitung 18 in das Mischrohr M gelangt und von dort das Ausströmventil 23 verlaßt. Durch den Strahldruck öffnet das Rückschlagventil bzw. hebt sich die Gummizunge 31 aus der Dichtstellung ab verbunden damit, daß der Wasser-Vorratsbehälter 1 bis Erreichen eines bestimmten Niveauspiegels gefüllt wird. Über die Ablauföffnung 13 verläßt das Spülwasser den Wasser-Vorratsbehälter 1 und wird über die Druckerhöhungspumpe zur Kammer geleitet. Nach beendetem Spülvorgang ist der Wasser-Vorratsbehälter 1 geleert, während der Wasseraufnahmeraum R des Dampferzeugers bis auf Höhe des Behälterbodens 2 gefüllt bleibt. Es tritt nun der Dampferzeuger 3 in Tätigkeit, wobei über die Heizwendel 34 das sie umgebende Wasser erhitzt wird bis zur Verdampfung desselben. Der Dampf füllt den Innenraum des Wasser-Vorratsbehälters 1 aus und verläßt über die Dampfaustrittsöffnung 36 den Wasser-Vorratsbehälter 1. Über ein nicht veranschaulichtes Dampfleitungsrohr gelangt der Dampf dann zur das Pflegegeschirr aufnehmenden Kammer und führt dort die thermische Desinfektion durch. Gleichzeitig werden eventuell im Wasser-Vorratsbehälter befindliche Bakterien durch den Dampf abgetötet. Unterschreitet der Niveauspiegel der Flüssigkeit den Temperaturfühler 35, führt dies über eine nicht veranschaulichte Steuereinrichtung zu einem Abschalten der Heizwendel 34. Entweder wird hierdurch die Desinfektion stillgesetzt oder es wird erneut Wasser zugeführt, um den Wasseraufnameraum R des Dampferzeugers 3 zu befüllen.

Während der Arbeit des Dampferzeugers 3 wird durch den Wasserdampf der im Behälterinneren befindliche Druck erhöht verbunden damit, daß die Gummizunge 31 in ihre Verschlußlage zur Wasserzulauföffnung 25 gezwungen wird.

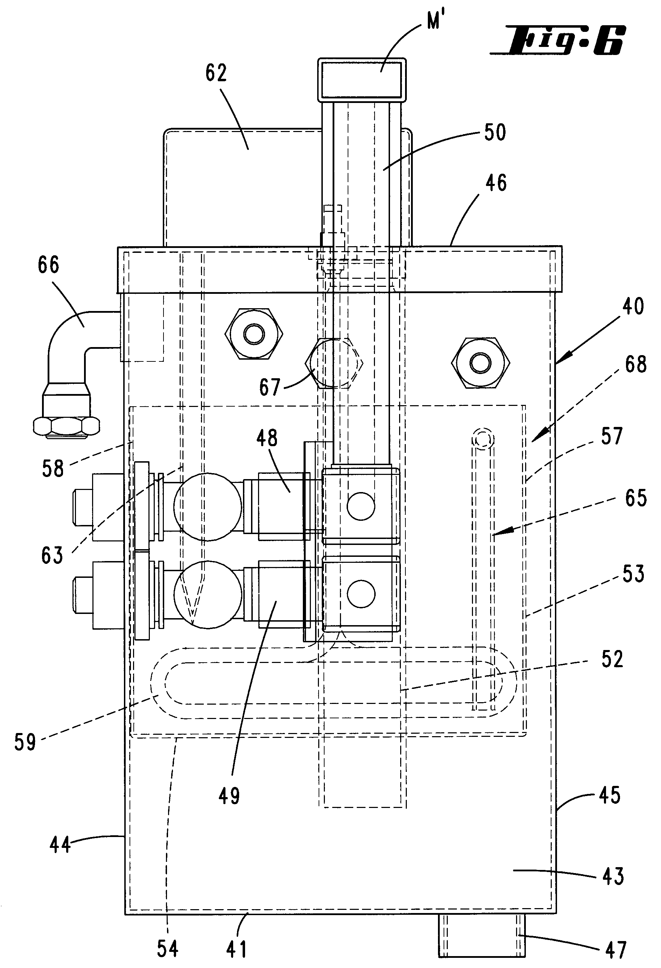

Der in den Fig. 5 bis 7 dargestellte Wasser-Vorratsbehälter 40 ist quaderförmig gestaltet. Er besitzt einen Behälterboden 41 mit von diesem in Aufwärtsrichtung ausgehenden Wandungen 42, 43, 44 und 45. Ein Deckel 46 überfängt das obere Ende des Wasser-Vorratsbehälters 40 und verschließt diesen. Der Behälterboden 41 ist mit einem Anschlußstutzen 47 ausgestattet, der seinerseits mit einer nicht veranschaulichten Pumpe in Verbindung steht, so daß über den Aßschlußstutzen 47 im Wasser-Vorratsbehälter 40 das Wasser zum Spülen entnommen werden kann.

Die Wandung 43 des Wasser-Vorratsbehälters 40 trägt die Zulaufstutzen 48, 49 für Kalt- und Warmwasser, welchen Zulaufstutzen jeweils ebenfalls ein nicht näher bezeichnetes Magnetventil zugeordnet ist. In Leitungsverbindung stehen die Zulaufstutzen 48, 49 in Verbindung mit einer Zulaufleitung 50, dessen oberes Ende in ein Mischrohr M' mündet. Dieses verläuft etwa horizontal und trägt unterseitig ein Ausströmventil 51. Dieses ist wie bei der ersten Ausführungsform ebenfalls mit nicht veranschaulichten Strahlleitkörpern zur Bildung eines Wasserstrahls ausgestattet.

In fluchtender Anordnung zum Ausströmventil 51 trägt der Deckel 46 ein zum Ausströmventil 51 hin offenes Tauchrohr 52, dessen unteres Ende nahe oberhalb des Behälterbodens 41 endet. Über dieses Tauchrohr 52 erfolgt das Befüllen des Wasser-Vorratsbehälters mit dem Spülwasser.

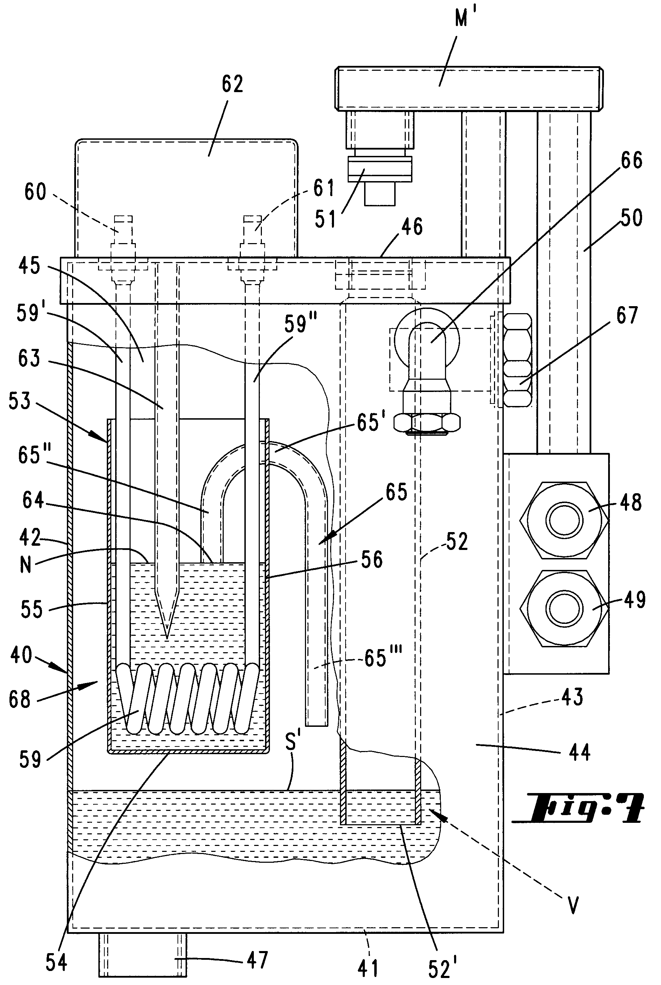

Innenseitig des Wasser-Vorratsbehälters 40 ist ein Innenbehälter 53 untergebracht. Dessen Boden 54 besitzt einen größeren Abstand zum Behälterboden 41 als das Austrittsende 52' des Tauchrohres 52 zum Behälterboden 41. Aus den Fig. 5 bis 7 ist ersichtlich, daß die Wandungen 55, 56, 57 des Innenbehälters 53 bis auf eine Wandung 58 beabstandet zu den Wänden 42, 43 und 45 des Wasser-Vorratsbehälters 40 angeordnet sind. Diese Wandung 58 dient zur Festlegung an der Wandung 44 des Wasser-Vorratsbehälters 40. Die Fig. 6 veranschaulicht, daß die Länge des Innenbehälters 53 nur geringfügig kleiner ist als die Innenseite des Wasser-Vorratsbehälters 40. Dies hat den Vorteil, daß die in den Innenbehälter 53 hineinragende Heizwendel 59 flach und lang ausgebildet sein kann verbunden mit einer großen Heizfläche. Die Enden 59', 59'' gehen vom Deckel 46 aus und stehen dort in Leitungsverbindung mit elektrischen Anschlüssen 60, 61. Überfangen sind diese von einer Schutzkappe 62. Es ist ersichtlich, daß die Heizwendel 59 dicht oberhalb des Bodens 54 des Innenbehälters 53 liegt.

Weiterhin ist am Deckel 46 ein in den Innenbehälter 53 hineinragender Temperaturfühler 63 vorgesehen.

Fig. 7 veranschaulicht, daß die Wandungen 55 bis 58 des Innenbehälters 53 ein Überlaufniveau N überragen. Dieses ist definiert durch die Mündungsöffnung 64 eines U-förmigen Hebers 65. Der U-Scheitel 65' verläuft unterhalb der Innenbehälter-Oberkante und ist durch die Wandung 56 hindurchgeführt und mit dieser in geeigneter Weise fest verbunden. Ferner veranschaulicht Fig. 7, daß das Überlaufniveau N etwa auf der Hälfte der Innenbehälterhöhe liegt.

Die Mündungsöffnung 64 befindet sich an dem kürzeren U-Schenkel 65'' des Hebers 65. Der andere U-Schenkel 65''', also der längere, ist etwa doppelt so lang wie der kürzere U-Schenkel 65'' und endet oberhalb des Bodens 54 des Innenbehälters 53.

Im oberen Bereich des Wasser-Vorratsbehälters 40 trägt die Wandung 44 einen Dampf-Austrittsstutzen 66, welcher in nicht veranschaulichter Weise mit der Desinfektionskammer in Verbindung steht.

Etwa auf gleicher Höhe zum Dampfaustrittsstutzen 66 ist in der Behälterwandung 43 eine nicht näher bezeichnete Inspektionsöffnung vorgesehen, welche durch einen Stopfen 67 verschliefbar ist.

Der Wasser-Vorratsbehälter gemäb dieser zweiten Ausführungsform arbeitet wie folgt:

Das Befüllen des Wasser-Vorratsbehälters 40 geschieht über die Zulaufstutzen 48, 49. Im Mischrohr M' findet die Vermischung statt, falls Warm- und Kaltwasser zugeführt werden. Das gemischte Wasser tritt über das Ausströmventil 51 in das Tauchrohr 52 ein und verläßt dieses über das Austrittsende 52' verbunden mit einem Ansteigen des Wasserspiegels. Gefüllt ist der Wasser-Vorratsbehälters 40, wenn der Wasserspiegel S etwa auf Höhe der durch den Stopfen 67 verschließbaren Inspektionsöffnung liegt. Mit dem Befüllen des Wasser-Vorratsbehälters findet auch das Befüllen des Innenbehälters 53 statt. In der Füllstellung erstrecken sich die oberen Randkanten der Wandungen 55 und 58 des Innenbehälters unterhalb des Wasserspiegels S. Definiert ist dieser Wasserspiegel S durch einen nicht dargestellten Niveauhöhenregler. Es kann nun zum Zwecke eines Spülprozesses im Wasser-Vorratsbehälter 40 das Wasser über den Anschlußstutzen 47 entnommen werden.

Ist die Desinfektionsphase mittels Dampf erwünscht, so wird der Wasser-Vorratsbehälter 40 so weit geleert, daß in diesem ein Restwasservolumen, wie in Fig. 7 veranschaulicht, verbleibt. Der Wasserspiegel S' fällt dann bis unterhalb des Bodens 54 ab, jedoch so, daß das untere Ende des Tauchrohres 52 noch in das im Behälter verbleibende Restwasservolumen eintaucht. Auf diese Weise wird von dem in das Restwasservolumen eintauchenden Tauchrohr ein syphonartiger Verschluß V geschaffen, welcher als Dampfaustritts-Widerstand dient.

Mit dem Entleeren des Wasser-Vorratsbehälters 40 findet über den Heber 65 ein teilweises Entleeren des Innenbehälters 53 statt, und zwar bis auf das Überlaufniveau N, welches, wie vorerwähnt wurde, durch die Mündungsöffnung 64 des U-förmigen Hebers 65 definiert ist.

Es kann nun der einen Dampferzeuger 68 bildende Innenbehälter 53 aktiviert werden, wobei der Heizwendel 59 elektrische Energie zugeführt wird. Hierdurch verdampft das im Innenbehälter 53 verbliebene Wasser, welcher Dampf über den Dampfaustrittsstutzen 66 den Wasser-Vorratsbehälter 40 verläßt. Aus dem deckelseitigen Ende des Tauchrohres 52 kann kein Dampf entweichen, da der syphonartige Verschluß V einen Dampfaustritts-Widerstand bildet.

Einem Verkalken des Innenraumes des Wasser-Vorratsbehälters 40 kann dadurch begegnet werden, daß permanent oder intervallartig dem Wasser-Vorratsbehälter Entkalkungsmittel zugegeben wird.

Alle offenbarten Merkmale sind erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

## Patentansprüche

1. Wasser-Vorratsbehälter (1) in einer Vorrichtung zum Spülen und/oder Desinfizieren von Pflegegeschirren, insbesondere Bett-Pflegegeschirr, mit einer das Pflegegeschirr aufnehmenden Kammer, welcher der Wasser-Vorratsbehälter (1) zulaufseitig vorgeordnet ist und mit einem mit dem Wasser des Wasser-Vorratsbehälters (1) gespeisten Dampferzeuger (3), dadurch gekennzeichnet, daß der Dampferzeuger (3) integraler Bestandteil des Wasser-Vorratsbehälters (1) ist.

2. Wasser-Vorratsbehälter nach Anspruch 1 oder insbesondere danach, dadurch gekennzeichnet, daß der Dampferzeuger (3) bodenseitig des Wasser-Vorratsbehälters (1) angeordnet ist.

3. Wasser-Vorratsbehälter nach einem oder mehreren der vorhergehenden Ansprüche oder insbesondere danach, dadurch gekennzeichnet, daß der Dampferzeuger (3) als bodenseitiger Fortsatz gestaltet ist.

4. Wasser-Vorratsbehälter nach einem oder mehreren der vorhergehenden Ansprüche oder insbesondere danach, dadurch gekennzeichnet, daß die Ablauföffnung (13) des Wasser-Vorratsbehälters (1) auf einer das Füllniveau des Dampferzeugers (3) definierenden Höhe liegt.

5. Wasser-Vorratsbehälter nach einem oder mehreren der vorhergehenden Ansprüche oder insbesondere danach, dadurch gekennzeichnet, daß die Dampfaustrittsöffnung (36) im oberen oder Deckelbereich des Wasser-Vorratsbehälters (1) liegt.

6. Wasser-Vorratsbehälter nach einem oder mehreren der vorhergehenden Ansprüche oder insbesondere danach, dadurch gekennzeichnet, daß die Wasserzulauföffnung (25) des Deckels (10) einen Dampfaustritts-Widerstand besitzt.

7. Wasser-Vorratsbehälter nach einem oder mehreren der vorhergehenden Ansprüche oder insbesondere danach, gekennzeichnet durch eine auf die Wasserzulauföffnung (25) gerichtete Wasserstrahl-Anstrittsmündung einer Wasserversoruungsleitung und ein die Wasserzulauföffnung (25) verschließendes Rückschlagventil (31), welches durch den Strahldruck öffenbar ist.

8. Wasser-Vorratsbehälter nach einem oder mehreren der vorhergehenden Ansprüche oder insbesondere danach, dadurch gekennzeichnet, daß das Rückschlagventil (31) als Gummizunge gestaltet ist.

9. Wasser-Vorratsbehälter nach einem oder mehreren der vorhergehenden Ansprüche oder insbesondere danach, dadurch gekennzeichnet, das, der Dampferzeuger (3) einen Wasseraufnahmeraum (R) ausbildet mit bodenseitiger Heizwendel (34), mit einem etwa auf halber Höhe des Wasseraufnahmeraumes (R) oberhalb der Heizwendel (34) angeordneten Temperaturfühler (35).

10. Wasser-Vorratsbehälter nach einem oder mehreren der vorhergehenden Ansprüche oder insbesondere danach, dadurch gekennzeichnet, das, der Dampfaustritts-Widerstand von einem syphonartigen Verschluß (V) ausgebildet ist.

11. Wasser-Vorratsbehälter nach einem oder mehreren der vorhergehenden Ansprüche oder insbesondere danach, dadurch gekennzeichnet, das, der syphonartige Verschluß (V) ein vom Deckel (46) bis unmittelbar über den Behälterboden (41) reichendes Tauchrohr (52) aufweist, welches in der Dampferzeugungsphase in den Wasserspiegel (S') eines im Behälter (40) verbleibenden Restwasservolumens eintaucht.

12. Wasser-Vorratsbehälter nach einem oder mehreren der vorhergehenden Ansprüche oder insbesondere danach, dadurch gekennzeichnet, das, der Dampferzeuger (68) einen sich oberhalb des Behälterbodens (41) erstreckenden Innenbehälter (53) besitzt.

13. Wasser-Vorratsbehälter nach einem oder mehreren der vorhergehenden Ansprüche oder insbesondere danach, dadurch gekennzeichnet, daß, der Boden (54) des Innenbehälters (53) oberhalb des Wasserspiegels (S') des Restwasservolumens liegt.

14. Wasser-Vorratsbehälter nach einem oder mehreren der vorhergehenden Ansprüche oder insbesondere danach, dadurch gekennzeichnet, daß, die Wandungen (55 bis 58) des Innenbehälters (53) ein Überlaufniveau (N) überragen.

15. Wasser-Vorratsbehälter nach einem oder mehreren der vorhergehenden Ansprüche oder insbesondere danach, dadurch gekennzeichnet, daß, das Überlaufniveau (N) durch die Mündungsöffnung (64) eines U-förmigen Hebers (65) definiert ist.

16. Wasser-Vorratsbehälter nach einem oder mehreren der vorhergehenden Ansprüche oder insbesondere danach, dadurch gekennzeichnet, daß, der U-Scheitel (65') unterhalb der Innenbehälterwand-Oberkante liegt.

17. Wasser-Vorratsbehälter nach einem oder mehreren der vorhergehenden Ansprüche oder insbesondere danach, dadurch gekennzeichnet, daß, das Überlaufniveau (N) etwa auf der Hälfte der Innenbehälterhöhe liegt.

18. Wasser-Vorratsbehälter nach einem oder mehreren der vorhergehenden Ansprüche oder insbesondere danach, dadurch gekennzeichnet, daß der Temperaturfühler (63) von oben in den Innenbehälter (53) ragt.

19. Wasser-Vorratsbehälter nach einem oder mehreren der vorhergehenden Ansprüche oder insbesondere danach, gekennzeichnet durch eine handverschließbare Inspektionsöffnung im oberen Bereich, insbesondere im Bereich des Wasserspiegels (S) des Wasser-Vorratsbehälters (40).

20. Wasser-Vorratsbehälter nach einem oder mehreren der vorhergehenden Ansprüche oder insbesondere danach, dadurch gekennzeichnet, daß die Länge des Innenbehälters (53) nur geringfügig kleiner ist als die Innenweite des Vorratsbehälters (40).

21. Wasser-Vorratsbehälter nach einem oder mehreren der vorhergehenden Ansprüche oder insbesondere danach, dadurch gekennzeichnet, daß die Wandungen (55, 56, 57) des Innenbehälters (53) bis auf eine Wandung (58) beabstandet zu den Wänden (42, 43, 45) des Wasser-Vorratsbehälters (40) angeordnet sind.
